# EUROPÄISCHE PATENTSCHRIFT

(11) **EP 4 090 504 B1**
(45) Veröffentlichungstag und Bekanntmachung des Hinweises auf die Patenterteilung: **18.02.2026**
(21) Anmeldenummer: 20845145.0
(22) Anmeldetag: 08.12.2020
(51) Int. Cl.: B26D 1/40, B26D 5/32, B65H 35/00, E04F 15/20, B65H 35/08, A61F 13/15, B65G 29/02

(54) **VORRICHTUNG UND VERFAHREN ZUM AUFBRINGEN EINES ZUSCHNITTS AUF EINEM TRÄGER**
DEVICE AND METHOD FOR DEPOSITING A BLANK ONTO A SUPPORT
DISPOSITIF ET PROCÉDÉ DE DÉPÔT D'UNE ÉBAUCHE SUR UN SUPPORT

(30) Priorität: 17.01.2020 DE 102020101049; 09.04.2020 DE 102020109983
(43) Veröffentlichungstag der Anmeldung: 23.11.2022
(73) Patentinhaber: Wächter Packautomatik GmbH & Co. KG, 33181 Bad Wünnenberg (DE)
(72) Erfinder: RASCHE, Thorsten, 33181 Bad Wünnenberg (DE)
(74) Vertreter: Ostermann, Thomas
(86) Internationale Anmeldenummer: PCT/DE2020/101035
(87) Internationale Veröffentlichungsnummer: WO 2021/143964

(56) Entgegenhaltungen:
- DE-A1- 10 301 837
- DE-A1- 102014 223 594
- KR-A- 20100 000 275

## Beschreibung

Die Erfindung betrifft eine Vorrichtung zum Aufbringen von Zuschnitten nach dem Oberbegriff des Patentanspruchs 1.

Ferner betrifft die Erfindung ein Verfahren zum Aufbringen von Zuschnitten nach dem Oberbegriff des Patentanspruchs 11.

Aus der DE 103 01 837 A1 ist eine Vorrichtung und ein Verfahren zum Aufbringen von Zuschnitten auf einen entlang einer Transportbahn bereitgestellten Träger bekannt. Die Vorrichtung weist eine Schneidwalze auf, die Messer zum Erzeugen von Zuschnitten aus einer Materialbahn aufweist, wobei die Materialbahn tangential der Schneidwalze zugeführt wird. In Förderrichtung nachgeordnet ist der Schneidwalze eine Vakuumtrommel, an der die Zuschnitte übergeben werden. Die Vakuumtrommel fördert die Zuschnitte weiter auf eine bezüglich einer Achse der Vakuumtrommel gegenüberliegenden Seite, an der die Zuschnitte von dem Träger übernommen und weitertransportiert werden. Der Träger ist als ein mit einer Klebeschicht versehenes Trägermaterial ausgebildet, das mit einer höheren Transportgeschwindigkeit betrieben wird als eine Umlaufgeschwindigkeit der Vakuumtrommel. Die Zuschnitte sind als Wundauflagen ausgebildet, die von der Vakuumwalze dem bandförmigen Träger übergeben werden. Nach nachfolgender Vereinzelung sind die Pflaster hergestellt. Da der Träger bandförmig ausgebildet ist, kommt es nicht auf eine bündige Anordnung zwischen dem Zuschnitt und dem Träger an.

Aus der DE 103 01 837 A1 ist ein Verfahren zur Herstellung von Pflastern bekannt, bei der ausgestanzte Wundauflagen auf eine Vakuumwalze übertragen werden. Mittels der Vakuumwalze werden die Wundauflagen auf ein bandförmiges Trägermaterial übergeben, wobei die Fördergeschwindigkeit des Trägermaterials höher ist als eine Umlaufgeschwindigkeit der Vakuumwalze. Eine bündige Übergabe an Träger ist nicht offenbart.

Aus der DE 10 2014 223 594 A1 ist eine Vorrichtung zum Aufbringen von Zuschnitten auf einen Träger bekannt. Die Vorrichtung umfasst eine Schneidwalze, die in einer Vakuumtrommel integriert ist. Ferner umfasst die Vorrichtung eine Dosiereinrichtung enthaltend eine Förderwalze und eine Steuereinrichtung zur Ansteuerung der Förderwalze. Eine Fördergeschwindigkeit der Förderwalze kann so eingestellt werden, dass die Förderwalze synchron mit einem Arbeitstakt der Schneidwalze verdreht wird. Auf diese Weise wird ein Schlupf zwischen den erzeugten Zuschnitten erzeugt, so dass die Zuschnitte nach und nach auf unterschiedliche, vorzugsweise zylinderförmige Träger übergeben werden können.

Aufgabe der vorliegenden Erfindung ist es, eine Vorrichtung und ein Verfahren zum Aufbringen von Zuschnitten auf entlang einer Transportbahn bereitgestellten Trägern derart anzugeben, dass die Zuschnitte bündig zu den auf der Transportbahn bereitgestellten Trägern übergeben werden.

Zur Lösung dieser Aufgabe weist die Erfindung die Merkmale des Patentanspruchs 1 auf.

Nach der Erfindung ist eine Dosiereinrichtung vorgesehen, mittels derer auf die Fördergeschwindigkeit einer Materialbahn, die einer Schneidwalze und einer Vakuumtrommel zugeführt wird, derart eingewirkt wird, dass die Zuschnitte unter Bildung eines Abstandes oder Versatzes zueinander von der Vakuumtrommel an entlang einer Transportbahn bereitgestellten vereinzelten Träger übergeben werden. Der Versatz oder der Abstand zwischen den Zuschnitten ist derart gewählt, dass die Zuschnitte unter bündigem Abschluss zu förderstromabwärtsseitig und förderstromaufwärtsseitig der Träger angeordneter Kanten derselben an die jeweiligen Träger übergeben werden können. Vorteilhaft können hierdurch aus einer bandförmigen Materialbahn versetzt zueinander angeordnete Zuschnitte erzeugt werden, die mittels der Vakuumtrommel unter bündigem Abschluss zu entlang einer Transportbahn bewegter Träger an dieselben sequentiell übergeben werden können. Wenn die Träger jeweils als Bodenbelagplatten und die Zuschnitte jeweils als Trittschallschutzfolien ausgebildet sind, können auf diese Weise Bodenbelagprodukte hergestellt werden, deren Schichten bündig zueinander angeordnet sind, ohne das nachträglich ein weiterer Schnitt erfolgen müsste. Ein ansonsten auftretender Versatz zwischen den Zuschnitten einerseits und der Bodenbelagplatte andererseits würde nach Verlegen der Bodenbelagprodukte zu kleinen Dellen führen, die unerwünscht sind.

Nach einer bevorzugten Ausführungsform der Erfindung weist die Dosiereinrichtung eine Dosierwalzenanordnung auf, die derart ansteuerbar ist, dass an der zwischen der Schneidwalze und der Vakuumtrommel eingespannten Materialbahn ein den Abstand der Zuschnitte zueinander vorgebender Schlupf erzeugt wird, der dem Abstand zwischen zweier sequenziell bereitgestellten Träger entspricht. Vorzugsweise wird eine Drehgeschwindigkeit der Dosierwalzenanordnung im Vergleich zur Vakuumtrommel und der Schneidwalze kurzzeitig verringert, so dass die Fördergeschwindigkeit der Transportbahn reduziert wird, was aufgrund der eingespannten Anordnung der Transportbahn zu dem gewünschten Schlupf führt. Die vergleichsweise geringere Drehgeschwindigkeit der Dosierwalzenanordnung und der bereits im Reibkontakt zwischen der Schneidwalze und der Vakuumtrommel befindlichen Transportbahn ermöglicht einen gezielt einstellbaren Schlupf, der zu einer Abstandsvergrößerung der an der Vakuumtrommel anhaftenden Zuschnitte führt. Der Abstand zwischen einem ersten Zuschnitt und einem in Förderrichtung vor demselben angeordneten zweiten Zuschnitt entspricht dem Abstand zwischen einem ersten Träger und einem in Transportrichtung vor demselben angeordneten zweiten Träger. Vorteilhaft kann auf einfache Weise eine bündige Übergabe bzw. Verbindung der Zuschnitte mit den sequenziell bereitgestellten Trägern erfolgen.

Nach einer bevorzugten Ausführungsform der Erfindung wird die Materialbahn tangential der Schneidwalze und der Vakuumtrommel zugeführt. Eine Verbindungslinie zwischen einer Drehachse der Vakuumtrommel und einer Drehachse der Schneidwalze weist einen Schneidpunkt der Vorrichtung auf, wobei im Schneidpunkt ein Messer der Schneidwalze zu einem Schnitt der Materialbahn führt. Da die Vakuumtrommel einen größeren Durchmesser aufweist als die Schneidwalze, ist ein Anlegepunkt der Materialbahn an der Vakuumtrommel in Förderrichtung hinter dem Schneidpunkt angeordnet. Der Schnitt an dem Schneidpunkt der Materialbahn erfolgt somit, während ein in Förderrichtung hinterer Teilabschnitt der Materialbahn schon an der Vakuumtrommel anhaftet. Auf diese Weise kann einfach durch Reduzierung der Drehgeschwindigkeit der Dosierwalzenanordnung ein relatives Verschieben des abgeschnittenen Randes der Materialbahn auf der Vakuumtrommel erzielt werden.

Nach einer Weiterbildung der Erfindung weist die Vorrichtung Förderansteuermittel auf, so dass eine Umfangsgeschwindigkeit der Vakuumtrommel synchron zu einer Transportgeschwindigkeit der Transportbahn ist. Vorteilhaft können hierdurch die Zuschnitte kontinuierlich an die Träger übergeben werden.

Nach einer Weiterbildung der Erfindung ist ein Durchmesser der Vakuumtrommel mindestens doppelt so groß wie ein Durchmesser der Schneidwalze. Je größer das Durchmesserverhältnis zwischen Vakuumtrommel und Schneidwalze ist, desto größer ist der Abstand zwischen dem Schneidpunkt und dem Anlegepunkt. Ist der Abstand zwischen dem Anlegepunkt und dem Schneidpunkt relativ groß, muss die Fördergeschwindigkeit der Dosierwalzenanordnung stark reduziert werden, um den gewünschten Schlupf zu erzeugen. Ist der Abstand zwischen dem Anlegepunkt und dem Schneidpunkt relativ klein, kann eine Reduzierung der Drehgeschwindigkeit der Dosierwalzenanordnung in unerwünschter Weise zu einem Ablösen der Materialbahn von der Vakuumtrommel erfolgen. Das Durchmesserverhältnis zwischen der Vakuumtrommel und der Schneidwalze ist derart gewählt, dass mit einer kurzzeitigen Drehgeschwindigkeitsreduzierung der Dosierwalzenanordnung der gewünschte Schlupf erzeugt wird.

Nach einer Weiterbildung der Erfindung weist die Vakuumtrommel eine eingelassene Gummileiste auf, die mit dem Messer der Schneidwalze zusammenwirkt, um an dem Schneidpunkt den gewünschten Schnitt der Materialbahn zu bewirken.

Zur Lösung der Aufgabe weist die Erfindung die Merkmale des Patentanspruchs 11 auf.

Der Vorteil des erfindungsgemäßen Verfahrens besteht darin, dass durch Ausübung einer Zugkraft auf einen an einer Vakuumtrommel anhaftenden Teilabschnitt einer Materialbahn eine vorgegebene Abstandsvergrößerung des so erzeugten vorderen Randes der Materialbahn zu einem in Förderrichtung vor demselben an der Vakuumtrommel anhaftenden hinteren Rand eines Zuschnitts erreicht werden kann. Mit weiterem Drehen der Vakuumtrommel und der Schneidwalze erhöht sich die Länge des anhaftenden Teilabschnitts. Mit Durchführung eines weiteren Schnitts wird ein hinterer Rand des Zuschnitts gebildet, so dass nun der Zuschnitt fertiggestellt ist und unter vollflächiger Anlage an der Vakuumtrommel in die Übergabeposition an den Träger bewegt wird. Vorteilhaft kann hierdurch auf einfache Weise eine definierte Einstellung eines Abstandes zwischen sequenziell an der Vakuumtrommel bereitgestellter Zuschnitte erfolgen.

Nach einer bevorzugten Ausführungsform der Erfindung erfolgt die auf die Materialbahn wirkende Zugkraft während eines Reibkontaktes des anhaftenden Teilabschnitts der Materialbahn zwischen der Vakuumtrommel und der Schneidwalze. Der vordere Teilabschnitt der Materialbahn ist somit zwischen der Vakuumtrommel und der Schneidwalze definiert eingespannt, so dass mittels der Zugkraft ein definierter Schlupf erzeugt wird, nachdem durch einen Schnitt die in Förderrichtung hintere Kante des Zuschnitts erzeugt worden ist. Der Schlupf wirkt somit auf den zwischen einem Schneidpunkt und in Förderrichtung hinteren Anlegepunkt verlaufenden Teilabschnitt der Materialbahn. Die Kraft wirkt somit auf einen Bereich des anhaftenden Teilabschnitts, der auf einer Verbindungslinie zwischen einer Drehachse der Vakuumtrommel und der Schneidwalze liegt, also jeweils tangential an der Vakuumtrommel und der Schneidwalze liegend. Insofern muss die Zugkraft größer sein als die radial auf den Teilabschnitt der Materialbahn wirkenden Reibkraft der Vakuumtrommel und der Schneidwalze.

Nach einer Weiterbildung der Erfindung ist die Dauer der Zugkraft abhängig von dem einzustellenden Schlupf, der zu dem gewünschten Abstand der sequenziell an der Vakuumtrommel anhaftenden Zuschnitte wirkt. Vorzugsweise wirkt die Zugkraft so lange auf die Materialbahn ein, dass der anhaftende Teilabschnitt um eine Wegstrecke zu einem bereits förderstromabwärts an der Vakuumtrommel anhaftenden Zuschnitt entgegen der Förderrichtung versetzt verschoben wird, Die Zuführung der Materialbahn zu der Vakuumtrommel und der Schneidwalze wird somit quasi um einen Zeitversatz verzögert, wobei ein so gebildeter vorderer Rand des an der Vakuumtrommel anhaftenden Teilabschnitts sich relativ zu der Vakuumtrommel bewegt.

Weitere Vorteile der Erfindung ergeben sich aus den weiteren Unteransprüchen.

Ein Ausführungsbeispiel der Erfindung wird nachfolgend anhand der Zeichnungen näher erläutert.

Es zeigen:
- Fig. 1: eine schematische Seitenansicht einer erfindungsgemäßen Vorrichtung zum Zeitpunkt eines durch Schnitt erzeugten hinteren Randes eines Zuschnitts,
- Fig. 2: die Seitenansicht der erfindungsgemäßen Vorrichtung zum Zeitpunkt einer Erzeugung einer Zugkraft auf eine Materialbahn, die einer Vakuumtrommel und einer Schneidwalze zugeführt wird,
- Fig. 3: die schematische Seitenansicht der Vorrichtung zum Zeitpunkt des Wegfalls der Zugkraft auf die Transportbahn, wobei ein Schlupf gebildet ist zwischen einem vorderen Rand der Materialbahn und dem in Förderrichtung vorderen Zuschnitt und
- Fig. 4: die schematische Seitenansicht der Vorrichtung zum Zeitpunkt eines weiteren Schnitts der Materialbahn zur Bildung eines hinteren Randes des Zuschnitts.

Eine Vorrichtung zum Aufbringen von Zuschnitten Z1, Z2, Z3 auf jeweils von entlang einer Transportbahn 1 bereitgestellten Träger 2 weist im Wesentlichen eine Vakuumtrommel 3, eine Schneidwalze 4 sowie eine Dosiereinrichtung 5 auf.

Die Träger 2 sind vorzugsweise als Bodenbelagplatten ausgebildet, die entlang der vorzugsweise linear bzw. geradlinig ausgebildeten Transportbahn 1 tangential der Vakuumtrommel 3 zugeführt werden. Die Träger 2 weisen jeweils eine in Transportrichtung 6 erstreckende Länge l_{T} auf. Die Träger 2 werden mit einer konstanten Transportgeschwindigkeit transportiert, wobei in Transportrichtung 6 hintereinander angeordnete Träger 2 einen Abstand a zueinander aufweisen. Eine in Transportrichtung 6 vordere Kante 7 des Trägers 2 weist stets den gleichen Abstand a zu einer hinteren Kante 8 des in Transportrichtung 6 vor demselben transportierten Träger 2 auf.

Es sind Förderansteuermittel vorgesehen, so dass die Träger 2 entlang der Transportbahn 1 tangential auf die Vakuumtrommel 3 mit einer solchen Transportgeschwindigkeit transportiert werden, die mit einer Umfangsgeschwindigkeit v_{V} der Vakuumtrommel 3 übereinstimmt.

Es sind Förderansteuermittel vorgesehen, so dass die Vakuumtrommel 3 mit der gleichen Drehgeschwindigkeit betrieben wird wie die Schneidwalze 4.

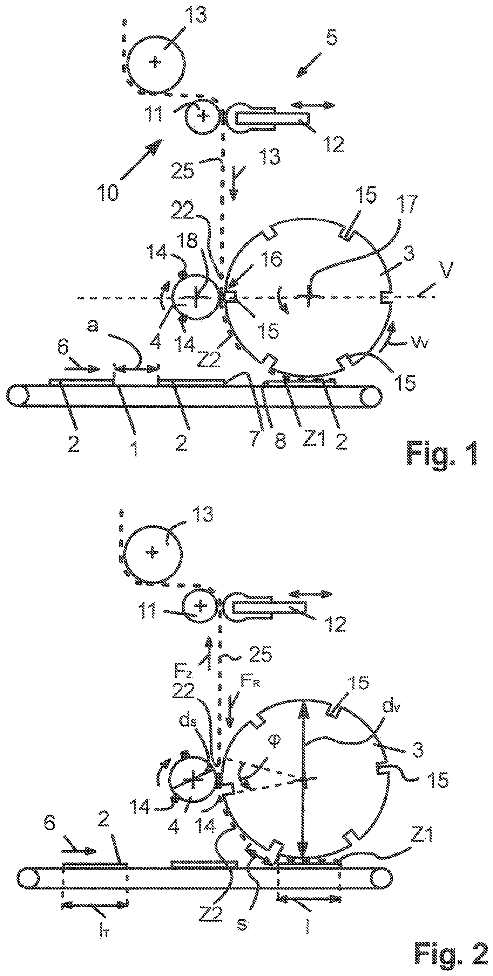

Wie aus Figur 1 zu ersehen ist, wird die Materialbahn 25 tangential der Vakuumtrommel 3 und der Schneidwalze 4 zugeführt. Die Materialbahn 25 wird zwischen der Schneidwalze 4 und der Vakuumtrommel 3 eingespannt. In Förderrichtung 9 hinter bzw. förderstromaufwärts der Vakuumtrommel 3 und der Schneidwalze 4 ist die Dosiereinrichtung 5 angeordnet. Die Dosiereinrichtung 5 umfasst eine Dosierwalzenanordnung 10 mit einer Dosierwalze 11 und einen auf eine Dosierwalze 11 wirkenden Linearaktor 12. Förderstromaufwärts zu der Dosierwalze 11 ist eine Umlenkrolle 13 angeordnet, an der die Materialbahn 25 um 90° umgelenkt wird, bevor sie auf die Dosierwalze 11 trifft. An der Dosierwalze 11 wird die Materialbahn 25 ebenfalls um 90° umgelenkt. Die Dosierwalze 11 ist derart angeordnet, dass die Materialbahn 25 tangential der Vakuumtrommel 3 und der Schneidwalze 4 zugeführt wird.

Im Folgenden wird an hand der Figuren 1 bis 4 die Herstellung eines Zuschnitts Z3 beschrieben.

In dem in Figur 1 dargestellten Zeitpunkt befinden sich die Vakuumtrommel 3 und die Schneidwalze 4 relativ zueinander in einer solchen Drehposition, dass ein Messer 14 der Schneidwalze 4 in eine an einer Umfangsfläche der Vakuumtrommel 3 angeordnete eingelassene Gummileiste 15 eingreift. In diesem Zeitpunkt erfolgt ein Schnitt der Transportbahn 1.

Die Vakuumtrommel 3 und die Schneidwalze 4 werden durch entsprechende Förderansteuermittel entgegengesetzte Drehrichtung verdreht. Während des Schnitts geben das Messer 14 und die Gummileiste 15 einen Schneidpunkt 16 vor, der auf einer gedachten Verbindungslinie V angeordnet ist, die eine Drehachse 17 der Vakuumtrommel 3 mit einer Drehachse 18 der Schneidwalze 4 verbindet.

Es ist ersichtlich, dass ein Durchmesser d_{V} der Vakuumtrommel 3 größer ist als ein Durchmesser d_{S} der Schneidwalze 4. Im vorliegenden Ausführungsbeispiel ist der Durchmesser d_{V} der Vakuumtrommel 3 mehr als doppelt so groß wie ein Durchmesser d_{S} der Schneidwalze 4; vorzugsweise ist der Durchmesser d_{V} der Vakuumtrommel 3 viermal so groß wie der Durchmesser d_{S} der Schneidwalze 4.

Aus Figur 1 ist ersichtlich, dass mit dem Schnitt der Materialbahn 25 an dem Schneidpunkt 16 ein in förderstromabwärts angeordneter hinterer Rand 19 des bereits vollständig an der Vakuumtrommel 3 anhaftenden Zuschnitts Z2 und zugleich ein förderstromabwärts angeordneter vorderer Rand 20 des zu erzeugenden Zuschnitts Z3 gleichzeitig erzeugt wird. Der in Förderrichtung 9 vor dem Zuschnitt Z3 angeordnete Zuschnitt Z2 erstreckt sich unter vollständiger Anhaftung an der Vakuumtrommel 3 weiter bis zu einem um 90° zu dem Schneidpunkt 16 versetzt angeordneten Übergabepunkt 21 der Vakuumtrommel 3, wobei der Übergabepunkt 21 wie der Schneidpunkt 16 an der Umfangsfläche der Vakuumtrommel 3 angeordnet ist. An dem Übergabepunkt 21 werden die Zuschnitte Z1, Z2, Z3 an die Träger 2 übergeben. Eine Länge l_{Z} der Zuschnitte Z1, Z2, Z3 ist somit kleiner als ein Viertel der Umfangsfläche der Vakuumtrommel 3. Im Übrigen entspricht die Länge l_{Z} der Zuschnitte Z1, Z2, Z3 der Länge l_{T} der Träger 2.

Es versteht sich, dass die Vakuumtrommel 3 eine Vielzahl kleiner Luftkanäle aufweist, die senkrecht zur Oberfläche der Vakuumtrommel 3 eingelassen sind. Auf diese Weise werden die Zuschnitte Z1, Z2, Z3 bzw. ein an der Vakuumtrommel 3 anhaftender Teilabschnitt 22 der Materialbahn 25 an der Umfangsfläche der Vakuumtrommel 3 festgehalten.

Aus Figur 1 ist ersichtlich, dass zum Zeitpunkt des Schnitts bereits die Materialbahn 25 förderstromaufwärts zu dem Schneidpunkt 16 an der Vakuumtrommel 3 anhaftet, und zwar an einem Anlagepunkt 23 der Vakuumtrommel 3. Der Schneidpunkt 16 und der Anlagepunkt 23 an der Umfangsfläche der Vakuumtrommel 3 bilden einen spitzen Winkel φ. Der Anlagepunkt 23 ist in Förderrichtung 9 hinter dem Schneidpunkt 16 angeordnet und bildet den erstmaligen Berührungskontakt der Materialbahn 25 an der Vakuumtrommel 3.

Wie aus Figur 1 ersichtlich ist, befindet sich der in Förderrichtung 9 vor deren Zuschnitt Z2 angeordnete Zuschnitt Z1 bereits im Bereich des Übergabepunktes 21, wobei er bündig zu dem heranlaufenden Träger 2 übergeben wird. Zu diesem Zweck weisen die Träger 2 jeweils eine nicht dargestellte Klebeschicht auf, so dass die aufeinander bündig zugeführten Träger 2 und Zuschnitte Z1, Z2, Z3 förderstromabwärts und förderstromaufwärts bündig miteinander verbunden werden. Ein vorderer Rand 20 der Zuschnitte Z1, Z2, Z3 schließt bündig mit einer vorderen Kante 7 der Träger 2 ab. Ein hinterer Rand 19 der Zuschnitte Z1, Z2, Z3 schließt bündig mit der hinteren Kante 8 der jeweiligen Träger 2 ab.

Zum in Figur 1 dargestellten Zeitpunkt wird die Dosiereinrichtung 4 mittels Förderansteuermittel mit der gleichen Drehgeschwindigkeit angesteuert wie die Vakuumtrommel 3 und die Schneidwalze 4.

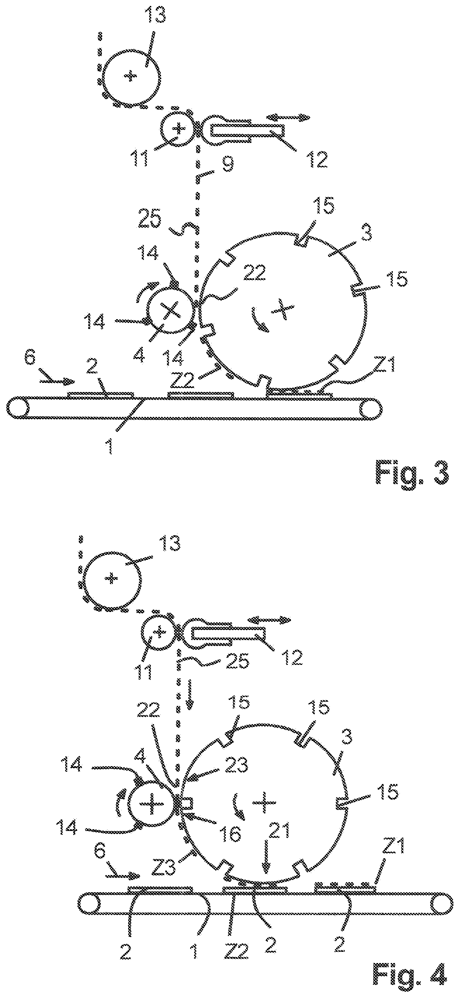

Nach Weiterdrehen der Vakuumtrommel 3 und der Schneidwalze 4 um einen spitzen Winkel in einer Drehposition gemäß Figur 2 wird der Linearaktor 12 betätigt, d. h. ausgefahren, so dass die Drehzahl der Dosierwalze 11 reduziert wird. Hierdurch entsteht eine Zugkraft F_{Z} entgegen der Förderrichtung 9, so dass ein Schlupf s auf den an der Vakuumtrommel 3 anhaftenden Teilabschnitt 22 entsteht. Die Fördergeschwindigkeit der Materialbahn 25 wird hierbei verringert, so dass sich ein Abstand des vorderen Randes 20 der Materialbahn 25 zu dem hinteren Rand 19 des Zuschnitts Z2 erhöht, und zwar so weit, bis der Schlupf s dem Abstand a zwischen zwei sequenziell hintereinander angeordneten Trägern 2 entspricht. Sobald der vorgegebene Schlupf s erreicht ist, wird der Linearaktor 12 deaktiviert, d. h. von der Dosierwalze 11 wegbewegt, mit der Folge, dass die Dosierwalze 11 wieder die gleiche Drehgeschwindigkeit aufweist wie die Vakuumtrommel 3 und die Schneidwalze 4. Die Materialbahn 25 kann somit unter Anhaften an der Vakuumtrommel 3 weiter zwischen der Vakuumtrommel 3 und der Schneidwalze 4 mit der gleichen Geschwindigkeit weiterbewegt werden unter Anhaftung an der Vakuumtrommel 3, s. Figur 3. Der vorgegebene Schlupf s ist somit eingestellt.

Sobald ein weiteres Messer 14 der Schneidwalze 4 mit einer weiteren Gummileiste 15 der Vakuumtrommel 3 auf der Verbindungslinie V angeordnet sind, erfolgt der nächste Schnitt, mittels dessen ein hinterer Rand 24 des Zuschnitts Z3 erzeugt wird. Der Zuschnitt Z3 in Figur 4 hat nun die gleiche Position wie der Zuschnitt Z2 in Figur 1. Durch Weiterverdrehung der Vakuumtrommel 3 und der Schneidwalze 4 wiederholen sich die bereits beschriebenen Schnitte und die Verzögerung der Materialbahn 25.

Der Schlupf s entspricht einer Wegstrecke, die einem Abstand zwischen dem hinteren Rand 19 des Zuschnitts Z2 und dem vorderen Rand 20 des Zuschnitts Z3 entspricht. Im vorliegenden Ausführungsbeispiel entspricht der Schlupf s annährend dem Abstand zwischen dem Anlagepunkt 23 und dem Schneidpunkt 16 der Vakuumtrommel 3. Der Winkel φ beträgt nur wenige Grad, beispielsweise 2° bis 5°.

Wesentlich für die Erzeugung des Schlupfes s ist, dass der vordere Teilabschnitt 22 der Transportbahn 1 in Kontakt mit der Vakuumtrommel 3 ist, während die Zugkraft F_{Z} ausgeübt wird.

Wenn die Materialbahn 25 nicht tangential zu der Vakuumtrommel 3 zugeführt wird, sondern in einem vergleichsweise kleineren Winkel zu der Umfangsfläche der Vakuumtrommel 3, kann die Zugkraft F_{Z} auch zeitlich früher ausgeübt werden, beispielsweise unmittelbar nach dem Schnitt der Materialbahn 25.

Im vorliegenden Ausführungsbeispiel ist der anhaftende Teilabschnitt 22 der Materialbahn 25 zwischen der Vakuumtrommel 3 und der Schneidwalze 4 eingespannt angeordnet, wobei ein Reibkontakt zwischen dem Teilabschnitt 22 einerseits und der Vakuumtrommel 3 und der Schneidwalze 4 andererseits besteht. Die Zugkraft F_{Z} muss somit größer sein als die auf den anhaftenden Teilabschnitt 22 wirkende Reibkraft F_{R}. Es versteht sich, dass die Länge l_{Z} der Zuschnitte Z1, Z2, Z3 kleiner ist als die Länge einer Umfangsfläche zwischen zwei in Förderrichtung hintereinander angeordneten Gummileisten 15 der Vakuumtrommel 3. Wenn die Zuschnitte Z1, Z2, Z3 und die Träger 2 länger ausgebildet sein sollen, müsste die Anzahl der Gummileisten 15 bzw. die Anzahl der Messer 14 an der Schneidwalze 4 entsprechend verringert werden.

Alternativ kann die Zugkraft F_{Z} auch auf den Teilabschnitt 22 einwirken, wenn der Teilabschnitt 22 die Vakuumtrommel 3 erst in dem Schneidpunkt 16 berührt. Hierbei wirkt der Zugkraft F_{Z} ausschließlich die durch den Reibkontakt zwischen der Vakuumtrommel 3 und der Schneidwalze 4 erzeugte Reibkraft F_{R} entgegen; und zwar ohne die zusätzliche Haftreibung des Teilabschnitts 22 an der Vakuumtrommel 3.

## Patentansprüche

1. Vorrichtung zum Aufbringen von Zuschnitten (Z1, Z2, Z3) auf einen Träger (2)
- mit einer Schneidwalze (4) enthaltend eine Anzahl von Messern (14) zum Erzeugen der Zuschnitte (Z1, Z2, Z3) aus einer Materialbahn (25),
- mit einer der Schneidwalze (4) zugeordneten Vakuumtrommel (3), mittels derer die Zuschnitte (Z1, Z2, Z3) unter Anhaften an der Vakuumtrommel (3) auf den in einer Transportbahn (1) bereitgestellten Träger (2) überführt wird, mit einer Dosiereinrichtung (5), die derart auf eine Fördergeschwindigkeit der Materialbahn (25) einwirkt, so dass die mittels der Schneidwalze (4) erzeugten Zuschnitte (Z1, Z2, Z3) um einen Abstand (s) versetzt zueinander an der Vakuumtrommel (3) anhaftend von derselben auf die entlang der Transportbahn (1) in einem Abstand (a) versetzt zueinander bereitgestellten vereinzelten Trägern (2) übergeben werden,
**dadurch gekennzeichnet, dass** die Dosiereinrichtung (5) eine Dosierwalze (11) und einen Linearaktor (12) zur Reduzierung einer Drehzahl der Drehwalze (11) aufweist, so dass eine Zugkraft (Fz) entgegen einer Förderrichtung (9) entsteht, und dass die Zuschnitte (Z1, Z2, Z3) jeweils den Trägern (2) übergeben werden, wobei die Zuschnitte (Z1, Z2, Z3) förderstromabwärts und förderstromaufwärts bündig zu den Trägern (2) angeordnet sind.

2. Vorrichtung nach Anspruch 1, **dadurch gekennzeichnet, dass** die Dosiereinrichtung (5) förderstromaufwärts zu der Schneidwalze (4) und der Vakuumtrommel (3) angeordnet ist und dass die Dosiereinrichtung (5) einen Dosierwalzenanordnung (10) aufweist, die derart ansteuerbar ist, dass die zwischen der Schneidwalze (4) und der Vakuumtrommel (3) eingespannte Materialbahn (25) um einen den Abstand der Zuschnitte (Z1, Z2, Z3) zueinander vorgebenden Schlupf (s) relativ zu einer Umfangsfläche der Vakuumtrommel (3) verzögert wird, der dem Abstand (a) zwischen zweier sequenziell bereitgestellter Träger (2) entspricht.

3. Vorrichtung nach Anspruch 1 oder 2, **dadurch gekennzeichnet, dass** die Dosiereinrichtung (5) derart angeordnet ist, dass die Materialbahn (25) tangential der Vakuumtrommel (3) und der Schneidwalze (4) zugeführt wird, und dass ein in einer Verbindungslinie (V) zwischen einer Drehachse (17) der Vakuumtrommel (3) und einer Drehachse (18) der Schneidwalze (4) angeordneter Schneidpunkt (16) in Förderrichtung (9) vor einem Anlagepunkt (23) der Materialbahn (25) an der Vakuumtrommel (3) angeordnet ist.

4. Vorrichtung nach einem der Ansprüche 1 bis 3, **dadurch gekennzeichnet, dass** Förderansteuermittel vorgesehen sind, so dass die Träger (2) mit einer zur Umfangsgeschwindigkeit der Vakuumtrommel (3) synchronen Transportgeschwindigkeit entlang der Transportbahn (1) transportiert werden.

5. Vorrichtung nach einem der Ansprüche 1 bis 4, **dadurch gekennzeichnet, dass** ein Durchmesser (dv) der Vakuumtrommel (3) mindestens doppelt so groß ist wie ein Durchmesser (d_{S}) der Schneidwalze (4).

6. Vorrichtung nach einem der Ansprüche 1 bis 5, **dadurch gekennzeichnet, dass** Förderansteuermittel vorgesehen sind, derart, dass die Vakuumtrommel (3) und die Schneidwalze (4) mit der gleichen Drehgeschwindigkeit betrieben werden.

7. Vorrichtung nach einem der Ansprüche 1 bis 6, **dadurch gekennzeichnet, dass** die Transportbahn (1) geradlinig und tangential auf die Vakuumtrommel (3) zulaufend verläuft.

8. Vorrichtung nach einem der Ansprüche 1 bis 7, **dadurch gekennzeichnet, dass** die Vakuumtrommel (3) eine eingelassene Gummileiste (15) aufweist, in der das Messer (14) der Schneidwalze (4) in der Schneidposition (16) eingreift zur Bildung des Schnitts der Materialbahn (25).

9. Vorrichtung nach einem der Ansprüche 1 bis 8, **dadurch gekennzeichnet, dass** die Träger (2) jeweils als eine Bodenbelagplatte und die Zuschnitte (Z1, Z2, Z3) jeweils als eine Trittschallschutzfolie ausgebildet sind.

10. Vorrichtung nach Anspruch 9, **dadurch gekennzeichnet, dass** die Bodenbelagplatte auf einer der Vakuumtrommel (3) zugewandten Flachseite mit einer Klebeschicht versehen ist.

11. Verfahren zum Aufbringen von Zuschnitten (Z1, Z2, Z3) auf entlang einer Transportbahn (1) bereitgestellten Träger (2), wobei eine Materialbahn (25) sequenziell einen Schnitt erfährt zum Erzeugen der Zuschnitte (Z1, Z2, Z3) und wobei die Zuschnitte (Z1, Z2, Z3) unter Anhaften an einer Vakuumtrommel (3) an den Träger (2) übergeben werden, dass die Materialbahn (25) zuerst der Vakuumtrommel (3) unter Bildung eines Anlagepunktes (23) an der Vakuumtrommel (3) und dann einem auf eine Verbindungslinie (V) zwischen einer Drehachse (d_{V}) der Vakuumtrommel (3) und eine Drehachse (ds) der Schneidwalze (4) liegenden Schneidpunkt (16) der Vakuumtrommel (3) und der Schneidwalze (4) zugeführt wird, an der ein der Vakuumtrommel (3) anhaftende Teilabschnitt (22) der Materialbahn (25) einen Schnitt erfährt unter Bildung eines vorderen Randes (20) des Zuschnitts (Z1, Z2, Z3), **dadurch gekennzeichnet, dass** der an der Vakuumtrommel (3) anhaftende Teilabschnitt (22) der Materialbahn (25) nach dem Schnitt derart eine Zugkraft (F_{Z}) erfährt, dass der abgeschnittene vordere Rand der Materialbahn (25) mittels Betätigen eines die Drehzahl einer Dosierwalze (11) reduzierenden Linearaktors (12) um einen Schlupf (s), der einem Abstand (a) zwischen zwei über die Transportbahn (1) synchron zur Vakuumtrommel (3) bereitgestellten Trägern (2) entspricht, entgegen der Drehrichtung der Vakuumtrommel (3) verschoben wird, und dass der an der Vakuumtrommel (3) anhaftende Teilabschnitt (22) der Materialbahn (25) den nächsten Schnitt an dem Schneidpunkt (16) zur Bildung eines hinteren Randes (19) desselben Zuschnitts (Z2) erfährt, so dass die Zuschnitte (Z1, Z2, Z3) jeweils den Trägern (2) übergeben werden.

12. Verfahren nach Anspruch 11, **dadurch gekennzeichnet, dass** die auf dem anhaftenden Teilabschnitt (22) der Materialbahn (25) wirkende Zugkraft (Fz) während eines Reibkontaktes des anhaftenden Teilabschnitts (22) der Materialbahn (25) zwischen der Vakuumtrommel (3) und der Schneidwalze (4) erfolgt.

13. Verfahren nach Anspruch 11 oder 12, **dadurch gekennzeichnet, dass** die Dauer der Zugkraft (F_{Z}) abhängig ist von dem einzustellenden Schlupf (s) der sequenziell an der Vakuumtrommel (3) anhaftenden Zuschnitte (Z1, Z2, Z3).

14. Verfahren nach einem der Ansprüche 11 bis 13, **dadurch gekennzeichnet, dass** die auf dem anhaftenden Teilabschnitt (22) der Materialbahn (25) wirkende Zugkraft (F_{Z}) so lange einwirkt, dass der anhaftende Teilabschnitt (22) der Materialbahn (25) um den Schlupf (s) zu einem förderstromabwärts an der Vakuumtrommel (3) anhaftenden Zuschnitt (Z2) versetzt entgegen der Förderrichtung (9) verschoben wird.

15. Verfahren nach einem der Ansprüche 11 bis 14, **dadurch gekennzeichnet, dass** die Schneidwalze (4) zumindest bei Kontakt eines umfangsseitig derselben abragenden Messers (14) mit einer zu der Vakuumtrommel (3) übereinstimmenden Drehzahl betrieben wird.

## Claims

1. A device for applying cutouts (Z1, Z2, Z3) to a support (2),
- comprising a cutting roller (4) containing a number of blades (14) for producing the cutouts (Z1, Z2, Z3) from a material web (25),
- comprising a vacuum drum (3) which is associated with the cutting roller (4) and by means of which the cutouts (Z1, Z2, Z3), while adhered to the vacuum drum (3), are transferred onto the support (2) provided in a transport path (1), and comprising a metering device (5) which influences a conveying speed of the material web (25) in such a way that the cutouts (Z1, Z2, Z3), produced by means of the cutting roller (4) and adhered to the vacuum drum (3) in a manner offset from one another by a distance (s), are transferred from said vacuum drum onto the separated supports (2) provided in a manner offset from one another by a distance (a) along the transport path (1),
**characterized in that** the metering device (5) includes a metering roller (11) and a linear actuator (12) for reducing a rotational speed of the metering roller (11) such that a tensile force (Fz) counter to a conveying direction (9) is produced, and **in that** the cutouts (Z1, Z2, Z3) are each transferred to the supports (2), the cutouts (Z1, Z2, Z3) being arranged flush with the supports (2) both on the downstream and upstream sides.

2. The device according to Claim 1, **characterized in that** the metering device (5) is arranged on the upstream side of the cutting roller (4) and the vacuum drum (3), and **in that** the metering device (5) includes a metering roller assembly (10) which can be controlled in such a way that the material web (25) clamped between the cutting roller (4) and the vacuum drum (3) is delayed by a slippage (s) relative to a circumferential surface of the vacuum drum (3), said slippage specifying the distance between the cutouts (Z1, Z2, Z3) and corresponding to the distance (a) between two sequentially provided supports (2).

3. The device according to Claim 1 or 2, **characterized in that** the metering device (5) is arranged in such a way that the material web (25) is supplied tangentially to the vacuum drum (3) and the cutting roller (4), and **in that** a cutting point (16) located on a connecting line (V) between an axis of rotation (17) of the vacuum drum (3) and an axis of rotation (18) of the cutting roller (4) is located downstream of a contact point (23) of the material web (25) with the vacuum drum (3) in the conveying direction (9).

4. The device according to any of Claims 1 to 3, **characterized in that** conveying actuating means are provided such that the supports (2) are transported along the transport path (1) at a transport speed synchronized with the circumferential speed of the vacuum drum (3).

5. The device according to any of Claims 1 to 4, **characterized in that** a diameter (dv) of the vacuum drum (3) is at least double a diameter (ds) of the cutting roller (4).

6. The device according to any of Claims 1 to 5, **characterized in that** conveying actuating means are provided such that the vacuum drum (3) and the cutting roller (4) are operated at the same rotational speed.

7. The device according to any of Claims 1 to 6, **characterized in that** the transport path (1) extends in a straight line and tangentially towards the vacuum drum (3).

8. The device according to any of Claims 1 to 7, **characterized in that** the vacuum drum (3) includes an inset rubber strip (15) into which the blade (14) of the cutting roller (4) reaches in the cutting position (16) in order to make the cut in the material web (25).

9. The device according to any of Claims 1 to 8, **characterized in that** the supports (2) are each designed as a floor covering board, and the cutouts (Z1, Z2, Z3) are each designed as an impact-sound insulating film.

10. The device according to Claim 9, **characterized in that** the floor covering board is provided with an adhesive layer on a flat side facing the vacuum drum (3).

11. A method for applying cutouts (Z1, Z2, Z3) to supports (2) provided along a transport path (1), wherein a material web (25) is sequentially cut in order to produce the cutouts (Z1, Z2, Z3) and wherein the cutouts (Z1, Z2, Z3), while adhered to a vacuum drum (3), are transferred to the supports (2), the material web (25) first being supplied to the vacuum drum (3) to form a contact point (23) with the vacuum drum (3) and then to a cutting point (16) of the vacuum drum (3) and the cutting roller (4), which cutting point is located on a connecting line (V) between an axis of rotation (dv) of the vacuum drum (3) and an axis of rotation (d_{S}) of the cutting roller (4) and at which a portion (22) of the material web (25) adhered to the vacuum drum (3) is cut to form a front edge (20) of the cutout (Z1, Z2, Z3), **characterized in that**, following the cut, the portion (22) of the material web (25) adhered to the vacuum drum (3) is subjected to a tensile force (Fz) in such a way that, by operating a linear actuator (12) that reduces the rotational speed of a metering roller (11), the cut front edge of the material web (25) is shifted by a slippage (s) counter to the rotational direction of the vacuum drum (3), said slippage corresponding to a distance (a) between two supports provided via the transport path (1) in synchronization with the vacuum drum (3), and **in that** the portion (22) of the material web (25) adhered to the vacuum drum (3) undergoes the next cut at the cutting point (16) to form a rear edge (19) of the same cutout (Z2), such that the cutouts (Z1, Z2, Z3) are each transferred to the supports (2).

12. The method according to Claim 11, **characterized in that** the tensile force (Fz) acting on the adhered portion (22) of the material web (25) occurs during frictional contact of the adhered portion (22) of the material web (25) between the vacuum drum (3) and the cutting roller (4).

13. The method according to Claim 11 or 12, **characterized in that** the duration of the tensile force (F_{Z}) is dependent on the slippage (s) to be set for the cutouts (Z1, Z2, Z3) sequentially adhered to the vacuum drum (3).

14. The method according to any of Claims 11 to 13, **characterized in that** the tensile force (Fz) acting on the adhered portion (22) of the material web (25) acts for such a duration that the adhered portion (22) of the material web (25) is shifted counter to the conveying direction (9) by the slippage (s) relative to a cutout (Z2) adhered to the vacuum drum (3) on the downstream side.

15. The method according to any of Claims 11 to 14, **characterized in that** the cutting roller (4), at least upon contact of a blade (14) that protrudes on the circumference thereof, is operated at a rotational speed that matches that of the vacuum drum (3).

## Revendications

1. Dispositif de dépôt de pièces découpées (Z1, Z2, Z3) sur un support (2)
- avec un rouleau de coupe (4) comportant un certain nombre de lames (14) pour produire les pièces découpées (Z1, Z2, Z3) à partir d'une bande de matériau (25),
- avec un tambour à vide (3) qui est associé au rouleau de coupe (4) et au moyen duquel les pièces découpées (Z1, Z2, Z3) sont transférées, en adhérant au tambour à vide (3), sur le support (2) mis à disposition dans une voie de transport (1), avec un dispositif de dosage (5) qui agit de telle manière sur une vitesse de transport de la bande de matériau (25) que les pièces découpées (Z1, Z2, Z3) produites au moyen du rouleau de coupe (4) sont transmises, adhérant sur le tambour à vide (3) de manière décalée entre elles selon un écart (s), depuis celui-ci sur les supports (2) individuels mis à disposition de manière décalée entre eux selon un écart (a) le long de la voie de transport (1),
**caractérisé en ce que** le dispositif de dosage (5) présente un rouleau de dosage (11) et un actionneur linéaire (12) destiné à réduire une vitesse de rotation du rouleau de dosage (11), de telle sorte qu'une force de traction (Fz) se produit à l'encontre de la direction de transport (9), et **en ce que** les pièces découpées (Z1, Z2, Z3) sont transmises respectivement aux supports (2), les pièces découpées (Z1, Z2, Z3) étant disposées affleurant les supports (2) en aval du flux de transport et en amont du flux de transport.

2. Dispositif selon la revendication 1, **caractérisé en ce que** le dispositif de dosage (5) est disposé en amont du flux de transport par rapport au rouleau de coupe (4) et au tambour à vide (3) et **en ce que** le dispositif de dosage (5) présente un ensemble rouleau de dosage (10) qui peut être commandé de telle manière que la bande de matériau (25) serrée entre le rouleau de coupe (4) et le tambour à vide (3) est retardée selon un glissement (s) définissant l'écart mutuel entre les pièces découpées (Z1, Z2, Z3) par rapport à une surface circonférentielle du tambour à vide (3), lequel correspond à l'écart (a) entre deux supports (2) mis à disposition de manière séquentielle.

3. Dispositif selon la revendication 1 ou 2, **caractérisé en ce que** le dispositif de dosage (5) est disposé de telle manière que la bande de matériau (25) est amenée de manière tangentielle au tambour à vide (3) et au rouleau de coupe (4), et **en ce qu'**un point de coupe (16) disposé dans une ligne de liaison (V) entre un axe de rotation (17) du tambour à vide (3) et un axe de rotation (18) du rouleau de coupe (4) est disposé, dans la direction de transport (9), avant un point d'appui (23) de la bande de matériau (25) sur le tambour à vide (3).

4. Dispositif selon l'une quelconque des revendications 1 à 3, **caractérisé en ce que** des moyens de commande de transport sont prévus de telle sorte que les supports (2) sont transportés le long de la voie de transport (1) avec une vitesse de transport synchrone par rapport à la vitesse circonférentielle du tambour à vide (3).

5. Dispositif selon l'une quelconque des revendications 1 à 4, **caractérisé en ce qu'**un diamètre (dv) du tambour à vide (3) est au moins deux fois plus grand qu'un diamètre (d_{S}) du rouleau de coupe (4).

6. Dispositif selon l'une quelconque des revendications 1 à 5, **caractérisé en ce que** des moyens de commande de transport sont prévus, de telle manière que le tambour à vide (3) et le rouleau de coupe (4) fonctionnent avec la même vitesse de rotation.

7. Dispositif selon l'une quelconque des revendications 1 à 6, **caractérisé en ce que** la voie de transport (1) s'étend de manière rectiligne et rejoint le tambour à vide (3) de manière tangentielle.

8. Dispositif selon l'une quelconque des revendications 1 à 7, **caractérisé en ce que** le tambour à vide (3) présente un listeau en caoutchouc (15) encastré, dans lequel la lame (14) du rouleau de coupe (4) vient en prise dans la position de coupe (16) pour former la coupe de la bande de matériau (25).

9. Dispositif selon l'une quelconque des revendications 1 à 8, **caractérisé en ce que** les supports (2) sont réalisés chacun sous la forme d'une plaque de revêtement de sol et les pièces découpées (Z1, Z2, Z3) sont réalisées chacune sous la forme d'un film de protection contre les bruits d'impact.

10. Dispositif selon la revendication 9, **caractérisé en ce que** la plaque de revêtement de sol est pourvue d'une couche adhésive sur un côté plat orienté vers le tambour à vide (3).

11. Procédé de dépôt de pièces découpées (Z1, Z2, Z3) sur des supports (2) mis à disposition le long d'une voie de transport (1), dans lequel une bande de matériau (25) fait l'objet, de manière séquentielle, d'une coupe pour produire les pièces découpées (Z1, Z2, Z3) et dans lequel les pièces découpées (Z1, Z2, Z3) sont transmises, en adhérant au tambour à vide (3), sur le support (2), la bande de matériau (25) est amenée d'abord au tambour à vide (3) en formant un point d'appui (23) sur le tambour à vide (3) puis à un point de coupe (16), situé sur une ligne de liaison (V) entre un axe de rotation (dv) du tambour à vide (3) et un axe de rotation (d_{S}) du rouleau de coupe (4), du tambour à vide (3) et du rouleau de coupe (4), au niveau duquel un tronçon (22) adhérant au tambour à vide (3) de la bande de matériau (25) fait l'objet d'une coupe en formant un bord avant (20) de la pièce découpée (Z1, Z2, Z3), **caractérisé en ce que** le tronçon (22) adhérant au tambour à vide (3) de la bande de matériau (25) fait l'objet, après la coupe, d'une force de traction (F_{Z}) telle que le bord avant coupe de la bande de matériau (25) est décalé, par l'actionnement d'un actionneur linéaire (12) réduisant la vitesse de rotation d'un rouleau de dosage (11), selon un glissement (s), qui correspond à un écart (a) entre deux supports (2) mis à disposition de manière synchrone par rapport au tambour à vide (3) par le biais de la voie de transport (1), à l'encontre du sens de rotation du tambour à vide (3), et **en ce que** le tronçon (22) adhérant au tambour à vide (3) de la bande de matériau (25) fait l'objet de la coupe suivante au niveau du point de coupe (16) pour former un bord arrière (19) de la même pièce découpée (Z2) de telle sorte que les pièces découpées (Z1, Z2, Z3) sont transmises respectivement aux supports (2).

12. Procédé selon la revendication 11, **caractérisé en ce que** la force de traction (F_{Z}) agissant sur le tronçon (22) adhérant de la bande de matériau (25) se produit pendant un contact de frottement du tronçon (22) adhérant de la bande de matériau (25) entre le tambour à vide (3) et le rouleau de coupe (4).

13. Procédé selon la revendication 11 ou 12, **caractérisé en ce que** la durée de la force de traction (Fz) est dépendante du glissement (s) à régler des pièces découpées (Z1, Z2, Z3) adhérant de manière séquentielle au tambour à vide (3).

14. Procédé selon l'une quelconque des revendications 11 à 13, **caractérisé en ce que** la force de traction (Fz) agissant sur le tronçon (22) adhérant de la bande de matériau (25) agit aussi longtemps que le tronçon (22) adhérant de la bande de matériau (25) est décalé à l'encontre de la direction de transport (9) selon le glissement (s) par rapport à une pièce découpée (Z2) adhérant au tambour à vide (3) en aval du flux de transport.

15. Procédé selon l'une quelconque des revendications 11 à 14, **caractérisé en ce que** le rouleau de coupe (4) fonctionne au moins lors du contact d'une lame (14) faisant saillie depuis celui-ci côté circonférence avec une vitesse de rotation correspondant au tambour à vide (3).
